# EUROPÄISCHE PATENTANMELDUNG

(11) **EP 3 928 634 A1**
(43) Veröffentlichungstag der Anmeldung: **29.12.2021**
(21) Anmeldenummer: 20000392.9
(22) Anmeldetag: 30.10.2020
(51) Int. Cl.: A23L 3/04, A61L 2/18, B08B 3/08, C01B 11/02

(54) **ANLAGE UND VERFAHREN ZUM BEHANDELN VON LEBENSMITTELBEHÄLTNISSEN**

(30) Priorität: 26.06.2020 AT 602042020
(71) Anmelder: Red Bull GmbH, 5330 Fuschl am See (AT)
(72) Erfinder: Die Erfindernennung liegt noch nicht vor
(74) Vertreter: Ellmeyer, Wolfgang

(57) **Zusammenfassung**

Die Erfindung betrifft eine Anlage zum Behandeln von Lebensmittel-Behältnissen durch Berieselung mit einer Biozid-hältigen Behandlungsflüssigkeit in zumindest einer mit Berieselungsmitteln (B) zur Berieselung der Behältnisse ausgestatteten Behandlungszone (Z1-Z4), wobei die Anlage weiters Transportmittel zum Transportieren der Behältnisse durch die Behandlungszonen (Z1-Z4), einen Generator (G1) zur Herstellung einer Biozid-Lösung, zumindest eine Heizeinrichtung zum Erhitzen der Behandlungsflüssigkeit, Leitungen zum Zuführen der Biozid-Lösung vom Generator (G1) zu den Behandlungszonen (Z1-Z4), und gegebenenfalls zu zumindest einer weiteren Biozid-Verbrauchereinrichtung (BV1, BV2), Leitungen zum Zuführen der Behandlungsflüssigkeit zu den Berieselungsmitteln (B), sowie zur Steuerung der Flüssigkeitsströme erforderliche Ventile (V1-V7), regelbare Dosiereinrichtungen und Pumpen umfasst, mit dem Kennzeichen, dass
a) die Anlage zumindest einen weiteren Generator (G2) zur Herstellung der Biozid-Lösung umfasst und
b) die Generatoren (G1, G2) weder direkt noch indirekt miteinander, aber über entsprechende Zuleitungen mit sämtlichen Behandlungszonen (Z1-Z4) und jeglichen weiteren Biozid-Verbrauchereinrichtungen (BV1, BV2) verbunden oder verbindbar sind;
sowie ein Verfahren zum Betreiben einer solchen Anlage.

## Beschreibung

Die vorliegende Erfindung betrifft eine Anlage und ein Verfahren zum Behandeln von dicht verschlossenen Lebensmittelbehältnissen mit einer Behandlungsflüssigkeit.

### STAND DER TECHNIK

Die Haltbarmachung von Lebensmitteln in dicht verschlossenen Behältnissen, wie z.B. Obst- und Gemüsesäften oder-konserven, Limonaden und Energy Drinks, erfolgt üblicherweise durch Pasteurisierung. Dabei werden die Behältnisse kurzzeitig auf über 60°C bis maximal 100 °C (Hochpasteurisierung) erhitzt, um die vegetativen Phasen von Mikroorganismen abzutöten. Dies stellt besonders im Vergleich zu der bei über 100 °C erfolgenden Sterilisation ein sehr schonendes Verfahren dar, bei dem die Konsistenz und die Inhaltsstoffe des Lebensmittels und somit auch deren Geschmack kaum beeinträchtigt werden.

Eine solche Pasteurisierung wird üblicherweise durch Berieseln mit einer entsprechend vorerhitzten wässrigen Behandlungsflüssigkeit in unterschiedlich, z. B. als Tunnelpasteur, Plattenpasteur oder Kammerpasteur, ausgelegten Pasteurisierungsanlagen durchgeführt. Aufgrund der kontinuierlichen Rezyklierung und Wiederverwendung der wässrigen Behandlungsflüssigkeit wird diese mit der Zeit zunehmend verunreinigt, In Kombination mit den erhöhten Temperaturen und der hohen Luftfeuchtigkeit der Umgebungsluft fördert dies die Vermehrung von Mikroorganismen in der Behandlungsflüssigkeit selbst und kann deren Verkeimung bewirken, was zur Ausbildung von Biofilmen auf den zu pasteurisierenden Lebensmittel-Behältnissen führen kann. Zur Verhinderung einer solchen Verkeimung und Biofilmbildung wird der Behandlungsflüssigkeit üblicherweise Desinfektionsmittel oder Biozide, wie z.B. Chlordioxid, zugesetzt.

Aufgrund ihrer eingeschränkten Stabilität werden derartige in der Behandlungsflüssigkeit eingesetzte Biozidlösungen *in situ* in speziellen Biozid-Generatoren der Pasteurisierungsanlage hergestellt, im Falle von Chlordioxid beispielsweise durch das Chlor-Chlorit-, das Salzsäure-Chlorit- oder das Peroxodisulfat-Chlorit-Verfahren. Solche Anlagen und entsprechende Behandlungsverfahren werden in DE 10 2018 205 009 A1 und der zugehörigen WO 2019/192817 A2 sowie in einer derzeit noch unveröffentlichten europäischen Patentanmeldung der Anmelderin offenbart.

Nachteilig ist daran allerdings, dass bei Störungen oder auch der Wartung des Biozid-Generators oder der mit diesem verbundenen Zu- und Ableitungen die gesamte Pasteurisierungsanlage zwischenzeitlich stillgelegt werden muss, bis die jeweiligen Arbeiten daran abgeschlossen sind.

Ziel der Erfindung war daher, die bekannten Pasteurisierungs- und ähnliche Anlagen zur Behandlung von Lebensmittelbehältern zu verbessern, um solche Abschaltungen vermeiden zu können.

### OFFENBARUNG DER ERFINDUNG

Dieses Ziel erreicht die vorliegende Erfindung in einem ersten Aspekt durch Bereitstellung einer Anlage zum Behandeln von dicht verschlossenen Lebensmittel-Behältnissen durch Berieselung mit einer zumindest ein Biozid enthaltenden Behandlungsflüssigkeit in zumindest einer mit einer Vielzahl von Berieselungsmitteln zur Berieselung der Behältnisse ausgestatteten Behandlungszone, wobei die Anlage weiters Transportmittel zum Transportieren der Behältnisse durch die Behandlungszonen, einen Generator zur Herstellung einer Lösung des zumindest einen Biozids, zumindest eine Heizeinrichtung zum Erhitzen der Behandlungsflüssigkeit, Leitungen zum Zuführen der Biozid-Lösung vom Generator zu der zumindest einen Behandlungszone, und gegebenenfalls zu zumindest einer weiteren Biozid-Verbrauchereinrichtung, Leitungen zum Zuführen der Behandlungsflüssigkeit zu den Berieselungsmitteln, sowie jegliche zur Steuerung der Flüssigkeitsströme erforderliche Ventile, regelbare Dosiereinrichtungen und Pumpen umfasst, wobei die erfindungsgemäße Anlage dadurch gekennzeichnet ist, dass
a) die Anlage zumindest einen weiteren Generator zur Herstellung der Biozid-Lösung umfasst und
b) die Generatoren weder direkt noch indirekt miteinander, aber über entsprechende Zuleitungen mit sämtlichen Behandlungszonen und allen weiteren Biozid-Verbrauchereinrichtungen verbunden oder verbindbar sind.

Durch die Bereitstellung zumindest eines zweiten Generators zur Herstellung der Biozid-Lösung wird dafür gesorgt, dass bei einem Ausfall des bislang einzigen Generators der Betrieb der gesamten, üblicherweise kontinuierlich und mit hohem Durchsatz betriebenen Anlage gänzlich eingestellt werden muss.

Die vorliegende Erfindung stellt hiermit jedoch nicht einfach nur eine Anlage mit einem oder mehreren weiteren Generatoren bereit, die allesamt den jeweils darin erzeugten Strom an Biozid-Lösung in einen gemeinsamen Vorratsbehälter - der eine "indirekte Verbindung" zwischen den Generatoren darstellen würde - einleiten, aus dem in der Folge alle Behandlungszonen und sonstigen Biozid-Verbrauchereinrichtungen gespeist werden. Dies würde die eingangs erwähnte Problematik nämlich nicht lösen, da auch dieser einzige Vorratsbehälter ausfallen kann und außerdem ebenfalls regelmäßig gewartet werden muss, was wiederum eine zwischenzeitliche Abschaltung der Anlage zur Folge hätte.

Vielmehr sind gemäß vorliegender Erfindung sämtliche, also zumindest zwei, Generatoren der Anlage, ohne direkt oder indirekt miteinander verbunden zu sein, über entsprechende Zuleitungen mit sämtlichen Behandlungszonen und sonstigen Biozid-Verbrauchereinrichtungen verbunden oder verbindbar. Dadurch können diese auch bei einem Ausfall oder der Wartung eines Generators weiterhin mit der Biozid-Lösung versorgt werden, womit sich eine Abschaltung der Anlage gänzlich erübrigt und deren diesbezügliche Ausfallssicherheit gewährleistet wird.

Weiters können auf diese Weise sämtliche Behandlungszonen und weiteren Biozid-Verbrauchereinrichtungen wahlweise mit Biozid-Lösung aus nur einem oder aus mehreren Generatoren gleichzeitig versorgt werden, was verschiedene weitere Vorteile mit sich bringt. Beispielsweise kann jeweils nur ein Teil der Behandlungszonen für einen bestimmten Zeitraum mit Biozid-Lösung aus allen Generatoren versorgt werden, während die übrigen Behandlungszonen abgeschaltet werden können, um sie zu reinigen oder zu warten. Auf weitere Vorteile, darunter die Möglichkeit, in den einzelnen Generatoren jeweils Biozid-Lösungen mit unterschiedlichen Parametern herstellen zu können, wird nachstehend noch näher eingegangen.

Dass in einem Generator hergestellte Biozid-Lösung in einen anderen einströmt, wird schon alleine dadurch verhindert, dass die Biozid-Lösungen aus den Generatoren jeweils mittels einer integrierten (und in den Figuren daher nicht gesondert dargestellten) Pumpe mit ausreichendem Druck ausgestoßen wird. In bevorzugten Ausführungsformen der Erfindung ist darüber hinaus am Auslass jedes Generators eine Rückschlagarmatur, wie z.B. ein Rückschlagventil oder eine Rückschlag- oder Rückstauklappe, vorzugsweise ein Rückschlagventil, vorgesehen. Auf diese Wiese wird für den Fall einer Pumpenfehlfunktion nicht nur ein Rückfluss der vom jeweiligen Generator hergestellten Biozid-Lösung in Letzteren verhindert, sondern auch ein Einströmen von Biozid-Lösung, die in einem anderen Generator hergestellt wurde, gänzlich ausgeschlossen.

Unter "Biozid-Verbrauchereinrichtungen" sind hierin sämtliche Vorrichtungen, Einrichtungen und Komponenten der erfindungsgemäßen Anlage zu verstehen, denen zumindest gelegentlich oder auch kontinuierlich Biozid-Lösung zuzuführen ist, um Schadorganismen, insbesondere Mikroben und Pilze, zu bekämpfen. Wobei unter "bekämpfen" sowohl eine Desinfektion im engeren Sinne, also eine Reduktion der Anzahl an Keimen um den Faktor 10⁻⁵, als auch Reduktionen in geringerem Ausmaß sowie die Einschränkung ihrer Vermehrung, z.B. die Verhinderung der eingangs erwähnten Verkeimung oder Biofilmbildung, zu verstehen ist. Folglich sind natürlich auch die Behandlungszonen für die Behältnisse selbst "Biozid-Verbraucher", während weitere Biozid-Verbrauchereinrichtung beispielsweise aus Einrichtungen zum Spülen, Waschen, Befüllen und Verschließen der Behältnisse sowie zum Schmieren der Transportmittel, wie z.B. von Transportbändern ("Bandschmierung"), für die Behältnisse ausgewählt sein können.

Die von den Generatoren zu den Biozid-Verbrauchern führenden Zuleitungen sind vorzugsweise über Mischventile, also etwa Drei-, Vier- oder Mehrwegemischer, miteinander verbunden oder verbindbar, wodurch die Biozid-Lösung aus den einzelnen Generatoren wahlweise zu allen oder nur zu einem Teil der Biozid-Verbraucher geleitet werden kann. Zudem kann der Anlage, vorzugsweise bei Verwendung von Vierwegemischern, gleichzeitig Frischwasser zugeführt werden, um Wasserverluste während des Betriebs auszugleichen.

In weiteren bevorzugten Ausführungsformen der Erfindung ist die Behandlungsflüssigkeit eine wässrige Lösung des zumindest einen Biozids, und die Generatoren sind für die Herstellung einer wässrigen Lösung zumindest eines Biozids mit variabler Konzentration ausgelegt, die höher ist als die Biozid-Konzentration der Behandlungsflüssigkeit, und weisen jeweils Zuleitungen für eine oder mehrere Biozid-Vorläuferlösungen sowie gegebenenfalls für Frischwasser und sonstige Zusätze zu der erzeugten Biozid-Lösung.

Noch bevorzugter dienen die Generatoren zur Herstellung einer wässrigen Chlordioxid-Lösung, insbesondere nach dem Peroxodisulfat-Chlorit-Verfahren, und weisen daher jeweils Zuleitungen für wässrige Lösungen von Peroxodisulfat und von Chlorit aus entsprechenden Vorratsbehältern auf. Allerdings sind ist natürlich auch durch das Chlor-Chlorit- oder das Salzsäure-Chlorit-Verfahren hergestelltes Chlordioxid sowie andere geeignete Biozide, darunter auch Desinfektionsmittel, von der Erfindung umfasst.

In bevorzugten Ausführungsformen dient die erfindungsgemäße Anlage zur Pasteurisierung der in den Behältnissen eingeschlossenen Lebensmittel mittels Berieselung mit einer heißen Behandlungsflüssigkeit und umfasst zu diesem Zweck zumindest eine Heizeinrichtung zum Erhitzen der Behandlungsflüssigkeit auf über 60 °C, vorzugsweise auf zumindest 80 °C. Besonders bevorzugt sind die Behandlungszonen der erfindungsgemäßen Anlage Bestandteile eines herkömmlichen Tunnelpasteurs, Plattenpasteurs oder Kammerpasteurs, insbesondere eines Tunnelpasteurs.

Die erfindungsgemäße Anlage umfasst vorzugsweise weiters Messsensoren für die Temperaturen und/oder die Konzentrationen der Flüssigkeitsströme, die besonders bevorzugt jeweils mit einer Steuereinheit zur Steuerung der Heizeinrichtungen, Dosiereinrichtungen, Pumpen und/oder Ventile auf Basis der gemessenen Werte verbunden sind.

Wie zuvor erwähnt, können in einer erfindungsgemäßen Anlage in den einzelnen Generatoren Biozid-Lösungen mit unterschiedlichen Parametern, z.B. mit unterschiedlichen Temperaturen und/oder Biozid-Konzentrationen, aber auch unterschiedliche Mengen an Biozid-Lösung, hergestellt werden. Dadurch können für einen Teil der Behandlungszonen und sonstigen Biozid-Verbraucher, oder auch für alle, verschiedene Parameter der Biozid-Lösung variiert werden, ohne die Betriebsbedingungen der Generatoren, oder zumindest nur jene eines Generators, verändern zu müssen.

In weiteren besonders bevorzugten Ausführungsformen umfasst die Anlage weiters eine oder mehrere Mischkammern zum Vermischen der in zumindest einem Generator hergestellten Biozid-Lösung mit Frischwasser, um diese zu verdünnen, und/oder zum Vermischen von zumindest zwei von unterschiedlichen Generatoren hergestellten Biozid-Lösungen vorderen Eintritt in die jeweilige Behandlungszone oder sonstige Biozid-Verbrauchereinrichtung. Dies ermöglicht beispielsweise für den Fall der Herstellung von Biozid-Lösungen mit unterschiedlichen Temperaturen und/oder Biozid-Konzentrationen oder von unterschiedlichen Biozid-Lösung-Volumenströmen in verschiedenen Generatoren, dass die Biozid-Lösung vor der Zufuhr zu den Biozid-Verbrauchern auf einen gewünschten Wert (Temperatur, Konzentration, Menge) gebracht werden kann, ohne dass in allen Generatoren eine Biozid-Lösung mit genau diesem Wert produziert zu werden braucht. Durch Zufuhr von Frischwasser in eine solche Mischkammer kann zudem die während der Behandlung der Behältnisse durch Verdampfung verbrauchte Flüssigkeitsmenge aufgefüllt werden - insbesondere in Ausführungsformen, bei denen die verbrauchte Behandlungsflüssigkeit gesammelt und rezykliert wird.

Mischkammern können beispielsweise anstelle von oben beschriebenen Mischventilen, insbesondere anstelle von Vier- oder Mehrwegemischem vorgesehen werden, um eine gründlichere Durchmischung mehrerer Ströme der Biozid-Lösung und/oder eines oder mehrerer Biozid-Lösungsströme mit Frischwasser zu gewährleisten. Unter Mischkammern sind hierin jedenfalls keine Sammel- oder Pufferbehälter oder Reservoire zu verstehen, die ebenfalls als indirekte Verbindungen zwischen Generatoren angesehen werden könnten. Vielmehr werden die Mischkammern gemäß vorliegender Erfindung dynamisch betrieben, d.h. die Summe der pro Zeiteinheit eingehenden Flüssigkeitsströme ist gleich jener der ausgehenden.

In weiteren bevorzugten Ausführungsformen der erfindungsgemäßen Anlage umfasst diese daher weiters Folgendes:
eine oder mehrere Zuleitungen für Frischwasser, die gegebenenfalls mit einem Frischwasserreservoir verbunden sind; und/oder
Mittel zum Sammeln und gegebenenfalls auch zum Rezyklieren von verbrauchter Behandlungsflüssigkeit; und/oder
weitere Heiz- oder Kühleinrichtungen; und/oder
Mittel zum Absaugen der die Behandlungszonen umgebenden Atmosphäre.

Auf diese Weise kann die Behandlungsanlage auf verschiedenste Weise an diverse gewünschte Bedingungen angepasst und somit optimiert werden. So kann etwa die Biozid-Konzentration der Behandlungsflüssigkeit durch Verdünnung eines oder mehrerer Ströme der Biozid-Lösung aus einem oder mehreren Generatoren, z.B. in einer oder mehreren Mischkammern oder auch direkt in Zuleitungen zu den Behandlungszonen, nachträglich durch Verdünnung reduziert werden.

Die Sammlung der verbrauchten Behandlungsflüssigkeit ist eine Standardprozedur bei einer Nutzung der Anlage für die Behandlung der Behältnisse in herkömmlichen Pasteuren, wie z.B. Tunnel-, Platten- oder Kammerpasteuren, und ihre Rezyklierung verringert die Kosten für den Betrieb der Anlage. Dabei wird in der Regel durch wiederholtes Berieseln der Behältnisse mit rezyklierter Behandlungsflüssigkeit auch deren Temperatur gesteuert, da sich beispielsweise eine bei Eintritt in eine Behandlungszone auf zumindest 80 °C vorerhitzte Behandlungsflüssigkeit bei jedem Kontakt mit einem Behältnis weiter abkühlt. Aus diesem Grund werden üblicherweise die Berieselungsmittel in manchen Bereichen einer Behandlungszone ausschließlich mit frischer, heißer Behandlungsflüssigkeit versorgt, während jene in anderen Bereichen mit unterhalb der Behältnisse, z.B. unterhalb eines Transportbands, gesammelter und bereits kühlerer Behandlungsflüssigkeit gespeist werden, was somit eine Rezyklierung derselben darstellt. Auf diese Weise kann beispielsweise ein Temperaturverlauf oder -gradient der Behältnisse innerhalb einer Behandlungszone eingestellt werden, ohne die einer Zone zugeführte Behandlungsflüssigkeit auf unterschiedliche Temperaturen erhitzen oder kühlen zu müssen.

Durch das Absaugen der Atmosphäre um die Behandlungszonen kann diese vor der Abgabe in die Umgebung gereinigt werden, was speziell bei Verwendung einer wässrigen Chlordioxid-Lösung als Biozidlösung erforderlich ist. Und ein Beheizen oder Kühlen von Biozid-Lösung oder von zur Ergänzung der Flüssigkeitsmenge eingespeistem Frischwasser ermöglicht eine (noch genauere) Steuerung der Temperatur in den Behandlungszonen oder in anderen Biozid-Verbrauchereinrichtungen.

In einem zweiten Aspekt stellt die vorliegende Erfindung auch ein Verfahren zum Behandeln von dicht verschlossenen Lebensmittel-Behältnissen durch Berieselung mit einer zumindest ein Biozid enthaltenden Behandlungsflüssigkeit unter Verwendung einer Anlage gemäß dem ersten Aspekt bereit, wobei das Verfahren die folgenden Schritte umfasst:
das Herstellen einer Lösung des zumindest einen Biozids in zumindest zwei Generatoren, die weder direkt noch indirekt miteinander verbunden sind;
das Leiten der Biozid-Lösung zu zumindest einer mit einer Vielzahl von Berieselungsmitteln ausgestatteten Behandlungszone, und gegebenenfalls zu zumindest einer weiteren Biozid-Verbrauchereinrichtung, wobei jeder Generator mit sämtlichen Behandlungszonen und jeglichen weiteren Biozid-Verbrauchereinrichtungen verbunden oder verbindbar ist;
das Vermischen der Behandlungsflüssigkeit mit der Biozid-Lösung; und
das Berieseln der Behältnisse mit der Behandlungsflüssigkeit über die Berieselungsmittel, während sie durch die Behandlungszonen transportiert werden.

Auf diese Weise sind natürlich dieselben Vorteile gegenüber dem Stand der Technik erzielbar, wie sie zuvor für die erfindungsgemäße Anlage beschrieben wurden: Das Verfahren ist kontinuierlich betreibbar, ohne dass bei einem Ausfall eines Generators die Anlage stillgelegt werden muss, und zudem können in den einzelnen Generatoren Biozid-Lösungen mit unterschiedlichen Parametern hergestellt werden. Vorzugsweise werden die Behältnisse dabei mit einer wässrigen Lösung des zumindest einen Biozids berieselt, und in den Generatoren wird eine wässrige Lösung des zumindest einen Biozids mit variabler Konzentration hergestellt, die höher ist als die Biozid-Konzentration der Behandlungsflüssigkeit, vorzugsweise eine wässrige Chlordioxid-Lösung nach dem Peroxodisulfat-Chlorit-Verfahren aus Peroxodisulfat und Chlorit.

In bevorzugten Ausführungsformen werden die in den Behältnissen eingeschlossenen Lebensmittel mit der Behandlungsflüssigkeit pasteurisiert, die zu diesem Zweck vor dem Berieseln der Behältnisse auf über 60 °C, vorzugsweise auf zumindest 80 °C, erhitzt wird. Weiters werden in bevorzugten Ausführungsformen die Temperatur und die Konzentrationen der Biozid-Lösungen und der Behandlungsflüssigkeit gemessen, und die Zuleitung der Biozid-Lösung und der Behandlungsflüssigkeit zu den Behandlungszonen und jeglichen weiteren Biozid-Verbrauchereinrichtungen wird anhand der gemessenen Werte gesteuert.

Wie ebenfalls erwähnt können in den Generatoren zumindest zeitweise wässrige Biozid-Lösungen mit unterschiedlichen Konzentrationen hergestellt werden, was die Behandlung der Behältnisse in den einzelnen Behandlungszonen und die Versorgung weiterer Biozid-Verbraucher flexibler gestaltet. Besonders bevorzugt werden Biozid-Lösungen aus verschiedenen Generatoren mit unterschiedlicher Biozid-Konzentration und/oder mit unterschiedlichem Volumenstömen vor dem Einleiten in eine Behandlungszone oder vor der Zufuhr zu einem anderen Biozid-Verbraucher miteinander vermischt, z.B. in einer oder mehreren Mischkammern. Dadurch kann die Biozid-Lösung auf einen vorbestimmten Konzentrations-Wert gebracht werden, oder es kann eine genauer regelbare Menge an Biozid-Lösung pro Zeiteinheit zu manchen oder allen Biozid-Verbrauchereinrichtungen zugeführt werden, ohne dass alle Generatoren eine Biozid-Lösung mit genau diesen Werten erzeugen müssen.

Weiters wird in bevorzugten Ausführungsformen des erfindungsgemäßen Verfahrens, in Analogie zur Anlage gemäß dem ersten Aspekt,
kontinuierlich oder intermittierend Frischwasser zugeführt; und/oder
verbrauchte Behandlungsflüssigkeit gesammelt und mitunter rezykliert; und/oder die Behandlungsflüssigkeit vor dem Eintritt in die Berieselungsmittel erhitzt oder gekühlt; und/oder
die die Behandlungszonen umgebende Atmosphäre abgesaugt,
wodurch jeweils wiederum die oben in Bezug auf die Anlage beschriebenen Vorteile erzielbar sind.

Weiters kann in bevorzugten Ausführungsformen des erfindungsgemäßen Verfahrens in die einzelnen Berieselungsmittel einer Behandlungszone Behandlungsflüssigkeit mit unterschiedlichen Temperaturen eingeleitet werden, wobei besonders bevorzugt in die in der Mitte einer Behandlungszone gelegenen Berieselungsmittel Behandlungsflüssigkeit mit der höchsten Temperatur eingeleitet wird. Insbesondere wird dabei die Behandlungsflüssigkeit vordem Eintritt in eine Behandlungszone auf zumindest 80 °C erhitzt und in die in der Mitte der Zone gelegenen Berieselungsmitteln eingeleitet, woraufhin sie sich während der Berieselung der Lebensmittel-Behältnisse abkühlt, unterhalb der Behältnisse gesammelt und rezykliert wird, indem sie in die am Anfang und am Ende der Zone gelegenen Berieselungsmittel eingeleitet wird. Zusätzlich kann dabei die in die am Anfang und am Ende der Zone gelegenen Berieselungsmittel eingeleitete Behandlungsflüssigkeit unterhalb der Behältnisse gesammelt und zur erneuten Berieselung von Behältnissen in diesen Bereichen rezykliert werden, wobei die Behandlungsflüssigkeit mit der in den Generatoren hergestellten Biozid-Lösung erst vermischt wird, sobald sie sich beim Berieseln der Behältnisse auf eine vorbestimmte Temperatur abgekühlt hat.

Auf diese Weise kann einerseits ein Temperaturverlauf oder -gradient der Behältnisse eingestellt werden, ohne die der Behandlungszone zugeführte Behandlungsflüssigkeit auf unterschiedliche Temperaturen erhitzen oder kühlen zu müssen, und gleichzeitig wird der Verbrauch an Biozid eingeschränkt. Beispielsweise kann die Behandlungsflüssigkeit durchwegs auf zumindest 80 °C vorgeheizt und den in der Mitte einer Behandlungszone gelegenen Berieselungsmitteln zugeführt werden, um die dort berieselten Behältnisse auf die höchste Temperatur zu erhitzen und die darin enthaltenen Lebensmitel zu pasteurisieren. Für eine vorzugsweise als Biozid eingesetzte Lösung von Chlordioxid wäre diese Temperatur jedoch zu hoch, das sich Chlordioxid bereits oberhalb von 45 °C zu zersetzen beginnt. Durch das Rezyklieren der Behandlungsflüssigkeit kühlt sich diese jedoch bei jedem Durchgang weiter ab, bis sie eine für den Kontakt mit Biozid geeignete Temperatur erreicht hat. Dabei ist zu berücksichtigen, dass die Biozid-Lösung deutlich kühler als die Behandlungsflüssigkeit ist, beispielsweise Raumtemperatur aufweist, so dass die Behandlungsflüssigkeit beim Vermischen mit der Biozid-Lösung weiter abgekühlt wird.

Diese mit Biozid versetzte Behandlungsflüssigkeit kann in der Folge zur Berieselung der Behältnisse über Berieselungsmittel am Anfang und am Ende der Behandlungszone, in Transportrichtung gesehen, eingesetzt werden. Auf diese Weise werden die z.B. mit einem Förderband transportierten Behältnisse nach ihrem Eintritt in eine Behandlungszone zunächst mit dem Gemisch aus abgekühlter Behandlungsflüssigkeit und Biozid-Lösung, das beispielsweise eine Temperatur von 30-40 °C aufweist, vorgewärmt und ihre Oberfläche mit Biozid behandelt. Anschließend wird ihr Inhalt mit heißer Behandlungsflüssigkeit, die auch ausschließlich aus heißem Wasser bestehen kann, pasteurisiert, wonach sie erneut mit kühlerer, Biozid enthaltender Behandlungsflüssigkeit berieselt werden, wobei die Temperatur der zu diesem Zweck rezyklierten Behandlungsflüssigkeit beim Kontakt mit den heißen Behältnissen wieder erhöht und jene der Behältnisse gleichzeitig gesenkt wird. Das heißt, die Behältnisse mit pasteurisiertem Inhalt werden vor ihrem Austritt aus der Behandlungszone bereits in einem gewünschten Ausmaß abgekühlt.

### KURZBESCHREIBUNG DER ZEICHNUNGEN

Die Erfindung wird nachstehend anhand von nichteinschränkenden bevorzugten Beispielen und unter Bezugnahme auf die beiliegenden Zeichnungen näher beschrieben, die Folgendes darstellen:
Fig. 1 ist eine schematische Ansicht einer relativ einfachen Ausführungsform einer Anlage und eines Verfahrens gemäß vorliegender Erfindung; und
die Fig. 2 und 3 sind schematische Ansichten weiterer bevorzugter Ausführungsformen der Erfindung mit etwas komplexerer Verschaltung.

### BEISPIELE

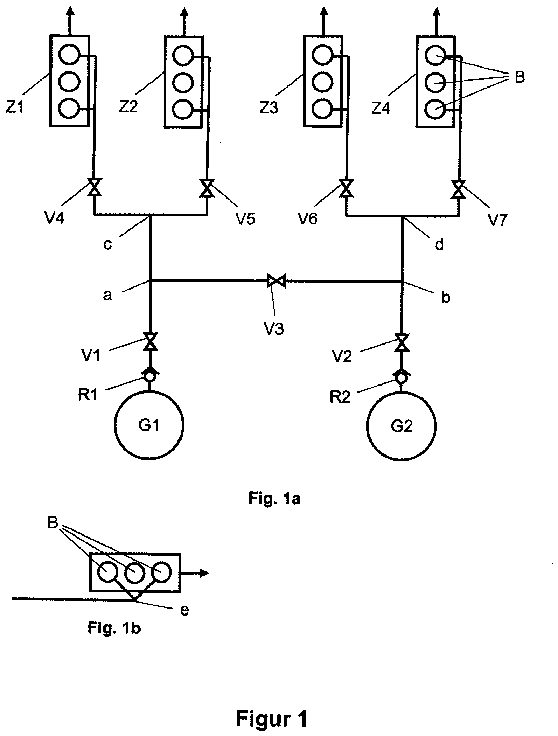

Wie oben erwähnt zeigt Fig. 1 schematisch eine vergleichsweise einfache Ausführungsform der Anlage und des Verfahrens der vorliegenden Erfindung, wobei allerdings nur die zur Verbindung der tatsächlich dargestellten Bauteile notwendigen Leitungen und Ventile eingezeichnet sind, nicht aber z.B. die für den Betrieb der Anlage ebenfalls erforderlichen Pumpen, sowie Heiz- und Kühleinrichtungen, weitere Ventile, steuerbare Dosiereinrichtungen, Regler und Steuereinheiten. Für den einschlägigen Fachmann ergeben sich die für einen reibungslosen Betrieb vorzusehenden Bauteile ganz selbstverständlich aus der Textbeschreibung. In Bezug auf die Beförderung der Flüssigkeiten durch die Anlage wird hierin beispielswiese angenommen, dass in jeden Bauteil, aus dem ein Flüssigkeitsstrom abgegeben wird, wie z.B. in Generatoren oder Vorratsbehältern, eine Pumpe mit der jeweils erforderlichen Pumpleistung integriert ist.

Die vorliegende Erfindung bezieht sich zudem in besonders bevorzugten Ausführungsformen auf den Betrieb von Tunnelpasteuranlagen, in denen eine zur Pasteurisierung von Lebensmitteln dienende heiße Behandlungsflüssigkeit zum Einsatz kommt, die mit Biozid-Lösung aus den Generatoren versetzt wird. Daher wird hierin weiters angenommen, dass die Anlage zumindest eine nicht dargestellte Heizeinrichtung umfasst, in der Wasser auf hohe Temperaturen, z.B. von 80-90 °C, erhitzt wird, um damit die Berieselungsmittel, insbesondere die Berieselungsmittel in der Mitte der jeweiligen Behandlungszonen, zu speisen und die darunter an den Berieselungsmitteln vorbei transportierten Lebensmittel-Behältnisse auf über 60 °C, vorzugsweise auf zumindest 70 °C, noch bevorzugter auf etwa 80 °C, zu erhitzen. Mitunter können auch Verdampfer eingesetzt werden, um die Behältnisse mit Wasserdampf auf die gewünschte Temperatur zu erhitzen, der an den Behältnissen kondensiert und wiederum unterhalb des Transportbandes gesammelt und rezykliert wird.

Weiters sei erwähnt, dass zur Vereinfachung und aus Gründen der Übersicht hierin nur Ausführungsformen mit zwei Generatoren konkret dargestellt und besprochen werden. Es versteht sich jedoch für den Fachmann von selbst, dass von der vorliegenden Erfindung auch Ausführungsformen mit mehr als zwei Generatoren umfasst sind, wobei die entsprechenden Bauteile jeweils analog zu verschalten sind.

In Fig. 1a ist nun ein erstes vereinfachtes Fließschema ausgehend von zwei Generatoren G1, G2 dargestellt, denen je eine Rückschlagarmatur R1, R2 nachgeschaltet ist. Dabei handelt es sich vorzugsweise um Rückschlagventile, z.B. federbelastete Kugelrückschlagventile, allerdings können auch Rückschlag- oder Rückstauklappen eingesetzt werden, um ein Rückströmen der Biozid-Lösung in den Generator und somit gleichzeitig das Einströmen von in einem anderen Generator hergestellter Biozid-Lösung wirksam zu verhindern, selbst wenn die integrierte Pumpe aufgrund einer Fehlfunktion oder dergleichen nicht den erforderlichen Druck erzeugen sollte.

Die in den Generatoren hergestellte Biozid-Lösung, bei der es sich insbesondere um eine wässrige Biozidlösung handelt, wird zu einer Vielzahl- hierin vier-Behandlungszonen Z1 bis Z4 gepumpt, die jeweils eine Vielzahl - hierin je drei - von durch Kreise O dargestellten Berieselungsmitteln B umfassen.

Es versteht sich von selbst, dass weder die Anzahl der Behandlungszonen noch jene der darin enthaltenen Berieselungsmittel darauf beschränkt ist. In der Praxis steht, wie zuvor angedeutet, jede der hierin besprochenen und dargestellten Behandlungszonen besonders bevorzugt für einen Tunnelpasteur, d.h. einen üblicherweise mehrere Meter langen Tunnel, in dem die Lebensmittel-Behältnisse, wie z.B. Getränkeflaschen oder -dosen, während sie darin verweilen bzw., z.B. auf einem Transportband, langsam hindurchbefördert werden, aus einer Vielzahl von Berieselungsmitteln von oben mit einer heißen Flüssigkeit berieselt werden. Nach dem Stand der Technik werden ebenfalls mehrere - gemäß der eingangs zitierten DE 10 2018 205 009 A1 beispielsweise je drei - Tunnelpasteure von einem Biozid-Generator mit Behandlungsflüssigkeit versorgt. Die maximale Anzahl hängt dabei vor allem von räumlichen und baulichen Gegebenheiten und von der Leistungsfähigkeit des Generators und der Pumpen ab und wird in der Regel kaum mehr als fünf betragen. Die Anzahl der Berieselungsmittel pro Behandlungszone bzw. Tunnelpasteur, bei denen es sich z.B. um Spritzdüsen oder Zerstäuber handelt, ist dabei natürlich ebenfalls von den obigen Bedingungen abhängig.

Den Behandlungszonen Z1 bis Z4 kann jeweils eine (nicht dargestellte) Dosiereinrichtung vorgeschaltet sein, die die Menge an der jeweiligen Behandlungszone zugeführten Biozid-Lösung steuert, also z.B. eine computergesteuerte Pumpe oder dergleichen, wobei diese Zufuhr kontinuierlich oder intermittierend ("Stoßdosierung") erfolgen kann.

In Fig. 1a ist in Flussrichtung unmittelbar nach den Rückschlagarmaturen R1 und R2 je ein Ventil V1 bzw. V2 vorgesehen, mittels dessen die Einspeisung von im jeweiligen Generator hergestellter Biozid-Lösung in die Anlage gänzlich unterbrochen werden kann. Danach ist in der hier dargestellten Ausführungsform die Einmündung der Biozid-Lösung aus dem jeweils anderen Generator zuführenden Leitung dargestellt, wobei die Verbindungsleitung ebenfalls ein Ventil, V3, umfasst, mit dem die Verbindung im Bedarfsfall unterbrochen werden kann.

In weiterer Folge verzweigen sich die von jedem Generator zu den Behandlungszonen führenden Leitungen, so dass bei geschlossenem Ventil V3 jeder der Generatoren G1 und G2 nur zwei Behandlungszonen, d.h. Z1 und Z2 bzw. Z3 und Z4, mit Behandlungsflüssigkeit versorgen kann. Jede der Leitungen zu den einzelnen Behandlungszonen Z1 bis Z4 umfasst ein weiteres Ventil, V4 bis V7, mittels dessen die Zufuhr von Behandlungsflüssigkeit zur jeweiligen Behandlungszone unterbrochen werden kann.

Die Berieselungsmittel B, der die Biozid-Lösung in der Folge, gegebenenfalls mittels einer steuerbaren Dosiereinrichtung einstellbar, zugeführt wird, sind vorzugswiese parallel geschaltet, wie in Fig. 1a dargestellt. Dadurch werden diese mit annähernd demselben Druck mit Biozid-Lösung versorgt. Besonders bevorzugt erfolgt die Zufuhr zu den Berieselungsmitteln B wie in Fig. 1b dargestellt, wodurch bei gleichem Leitungsvolumen derselbe Druck in allen Leitungen gewährleistet ist. An der mit "e" gekennzeichneten Position kann dabei beispielsweise jeweils eine computersteuerbare Dosiereinrichtung vorgesehen sein.

In den Fig. 1a und 1b wird zudem eine bevorzugte Ausführungsform der Erfindung gezeigt, bei der die aus den Generatoren zugeführte Biozid-Lösung nicht in alle Berieselungsmitteln B, sondern nur in jene am Anfang und am Ende der Behandlungszonen, hierin somit in das erste und das dritte Berieselungsmittel B, eingespeist wird. Dabei wird angenommen, dass die in der Mitte der Behandlungszonen gelegenen Berieselungsmittel B mit Behandlungsflüssigkeit mit der höchsten Temperatur berieselt (oder auch besprüht) werden, z.B. mit 80-90 °C heißem Wasser. Da sich beim Vermischen der Biozid-Lösung mit derartig heißem Wasser das Biozid, insbesondere Chlordioxid, zersetzen würde, erfolgt die Vermischung erst, nachdem sich das als Behandlungsflüssigkeit dienende Wasser durch Rezyklierung, d.h. Auffangen des an der Oberfläche der zu behandelnden Behältnisse herabgeronnenen Wassers unterhalb des Transportbands und erneutes Berieseln von Behältnissen am Anfang und am Ende der Behandlungszone, ausreichend abgekühlt hat.

Weiters können an den Verzweigungspositionen a bis d der Leitungen zusätzlich oder vorzugsweise alternativ zu den Ventilen V1 bis V7 Mehrwegventile vorgesehen werden. So kann etwa das Ventil V3 durch zwei Mehrwegventile an den Positionen a und b ersetzt werden, und/oder die Ventile V4 bis V7 können durch Mehrwegventile an den Positionen c und d ersetzt werden. Dies bietet zusätzlich den Vorteil, dass bei Verwendung von Vierwegemischern an der jeweiligen Position beispielsweise Frischwasser in die jeweilige Leitung eingeleitet werden kann, beispielsweise um Flüssigkeitsverluste auszugleichen oder die Biozid-Lösung erforderlichenfalls zu verdünnen. Allerdings ist die Einmündung einer Frischwasser oder dergleichen zuführenden Leitung nicht auf Mehrwegventile an diesen Positionen beschränkt, sondern kann auch an einer beliebigen anderen Position innerhalb der Anlage erfolgen.

Weiters kann beispielsweise anstelle mancher oder aller Mehrwegemischer jeweils eine dynamisch betriebene Mischkammer, wie zuvor beschrieben, vorgesehen werden, um die Durchmischung zu verbessern, was speziell im Fall von mehr als zwei zu vermischenden Strömen von Vorteil sein kann.

In der Praxis, z.B. in erfindungsgemäß besonders bevorzugten Tunnelpasteuren, wird die durch Vermischen der wässrigen Biozid-Lösung mit dem ausreichend abgekühlten Wasser gebildete Behandlungsflüssigkeit mehrere Male rezykliert und schließlich von den Behandlungszonen abgezogen, wie dies durch die Pfeile angedeutet ist, und - gegebenenfalls nach entsprechender Reinigung bzw. Aufbereitung - entweder entsorgt, erneut zur Berieselung von Behältnissen eingesetzt, oder aber anderen Biozid-Verbrauchern zugeführt.

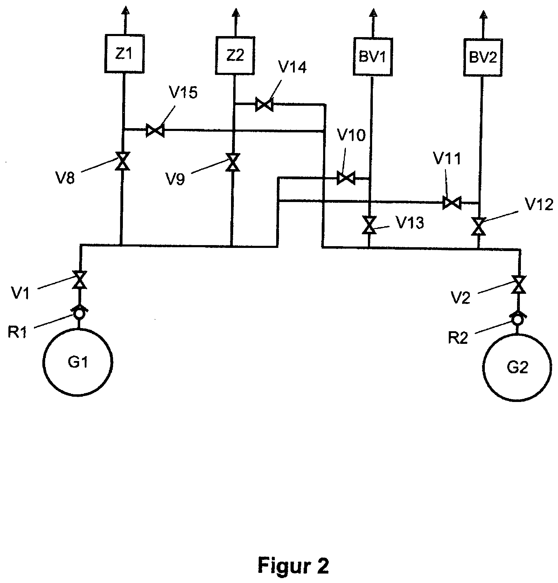

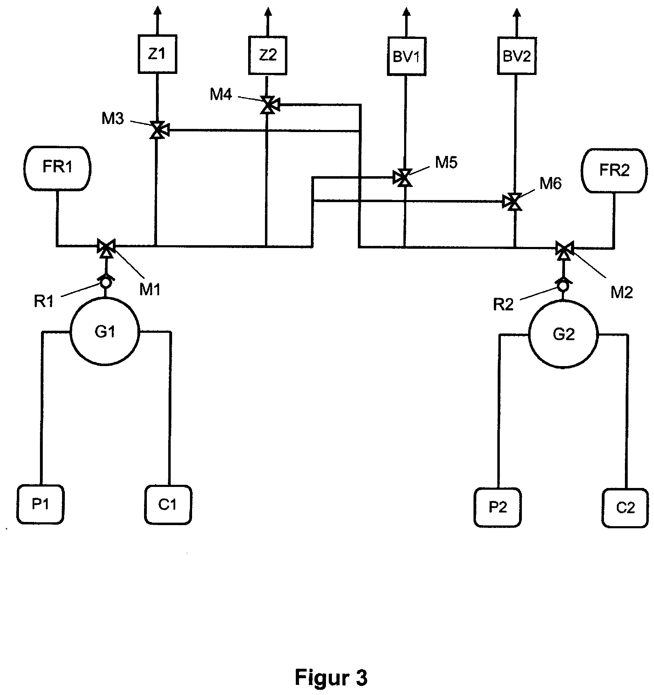

In Fig. 2 ist eine andere Ausführungsform der erfindungsgemäßen Anlage bzw. des erfindungsgemäßen Verfahrens wie in Fig. 1 dargestellt, bei der wiederum zwei Generatoren G1 und G2, nun allerdings nur zwei Behandlungszonen (z.B. Tunnelpasteure) Z1 und Z2 sowie zwei weitere Biozid-Verbrauchereinrichtungen BV1 und BV2 mit darin hergestellter Biozid-Lösung versorgen. Der Einfachkeit halber wird hier - wie auch in der folgenden Fig. 3 - auf die explizite Darstellung der einzelnen Berieselungsmittel innerhalb der Behandlungszonen verzichtet.

Als weitere Biozid-Verbraucher sind in herkümmlichen Anlagen zum Behandeln von Lebensmittel-Behältnissen vor allem Einrichtungen zum Spülen, Waschen, Befüllen und Verschließen der Behältnisse sowie solche zum Schmieren der Transportmittel, z.B. eines oder mehrerer Transportbänder, für die Behältnisse ("Bandschmierung") vorgesehen. Diese werden ebenfalls - kontinuierlich oder intermittierend - mit in den Generatoren hergestellter Biozid-Lösung, gegebenenfalls nach Verdünnung derselben mit Frischwasser oder einer sonstigen Aufbereitung, versorgt.

Der weitere Hauptunterschied zur Ausführungsform aus Fig. 1 liegt darin, dass die von den beiden Generatoren G1 und G2 zu den Behandlungszonen Z1 und Z2 und den beiden weiteren Biozid-Verbrauchern BV1 und BV2 zugeführte Biozid-Lösung in diesem Fall unmittelbar vor den jeweiligen Biozid-Verbrauchern Z1, Z2, BV1 und BV2 in die entsprechende Zuleitung eingespeist werden kann, so dass bei gleichzeitigem Betrieb beider Generatoren G1 und G2 die Zufuhr von Biozid-Lösung von einem davon auch nur für einen einzelnen Biozid-Verbraucher unterbrochen werden kann, während das bei der in Fig. 1 gezeigten Variante nur paarweise möglich ist. Das heißt, es kann für jeden Biozid-Verbraucher individuell eingestellt werden, ob er von einem oder von beiden - oder bei mehr als zwei Generatoren von mehreren oder von allen - mit Biozid-Lösung versorgt werden soll. Auf diese Weise kann eine genaue Regulierung der zu jedem Biozid-Verbraucher Z1, Z2, BV1 und BV2 zugeführten Biozid-Lösung erfolgen, auch wenn sich die in den einzelnen Generatoren hergestellten Biozid-Lösungsströme in manchen Parametern unterscheiden, z.B. wenn diese unterschiedliche Konzentrationen an Biozid, unterschiedliche Volumenströme oder auch unterschiedliche Temperaturen aufweisen.

Ansonsten gelten für die in Fig. 2 dargestellte Ausführungsform der erfindungsgemäßen Anlage bzw. des erfindungsgemäßen Verfahrens dieselben Optionen wie zuvor für Fig. 1 beschrieben. So können etwa auch in diesem Fall an allen Verzweigungspositionen von Leitungen zusätzlich oder alternativ zu den Ventilen V8 bis V15 Mehrwegeventile vorgesehen werden, es kann eine Zufuhr für Frischwasser erfolgen usw.

In Fig. 3 ist schließlich eine besonders bevorzugte Ausführungsform dargestellt, in der gegenüber jener in Fig. 2 die Ventile V1, V2 und V8 bis V15 tatsächlich jeweils durch Mehrwegemischer M1 bis M6 ersetzt sind. In den Zuleitungen zu den vier Biozid-Verbrauchern Z1, Z2, BV1 und BV2 sind anstatt der Ventile V8 bis V15 die Dreiwegemischer M3 bis M6 vorgesehen, und die auf die Rückschlagarmaturen R1 und R2 folgenden Ventile V1 und V2 wurden durch die Dreiwegemischer M1 und M2 ersetzt, in die zusätzlich noch jeweils eine Zuleitung für Frischwasser aus den Frischwasserreservoiren FR1 bzw. FR2 einmündet. Natürlich könnte jedoch auch in diesem Fall, wie oben in Bezug auf Fig. 1 erwähnt, auch in die Mehrwegemischer M3 bis M6 noch eine zusätzliche Frischwassereinspeisung erfolgen, falls sie statt als Dreiwege- als Vierwegemischer ausgeführt werden.

Darüber hinaus sind in Fig. 3 noch Vorratsbehälter für wässrige Lösungen von Peroxodisulfat, P1 und P2, bzw. von Chlorit, C1 und C2, dargestellt, die über entsprechende Zuleitungen die beiden Generatoren G1 und G2 mit diesen Biozid-Vorläuferlösungen versorgen, um darin die erfindungsgemäß als Biozid-Lösung besonders bevorzugte Chlordioxid-Lösung nach dem Peroxodisulfat-Chlorit-Verfahren herzustellen.

Alle weiteren oben beschriebenen Optionen gelten sinngemäß natürlich auch für die in Fig. 3 gezeigte Ausführungsform. Somit wird auch hier davon ausgegangen, dass die beiden Frischwasserreservoire FR1 und FR2 sowie die Vorratsbehälter für Biozid-Vorläuferlösungen P1, P2, C1 und C2 jeweils über eine integrierte Pumpe verfügen, und jedes Mehrwegeventil kann optional durch eine Mischkammer ersetzt werden.

Dem einschlägigen Fachmann auf dem Gebiet der Behandlung von Lebensmitteln und speziell von Getränken, insbesondere in Tunnelpasteuren, ist außerdem eine Reihe weiterer, für verschiedene Anwendungen oder Verfahrensführungen erforderlicher oder wünschenswerter Modifikationen und Ergänzungen bekannt, die folglich in Kombination mit den hierin hierin offenbarten Merkmalen der Erfindung ebenfalls unter den Schutz der beiliegenden Ansprüche fallen.

Die vorliegende Erfindung stellt somit eine neue Anlage und ein neues Verfahren zur Behandlung von dicht verschlossenen Lebensmittel-Behältnissen durch Berieselung mit einer Biozid-hältigen Behandlungsflüssigkeit bereit, die gegenüber dem Stand der Technik eine höhere Ausfallssicherheit sowie eine höhere Flexibilität der Behandlung selbst gewährleisten.

## Patentansprüche

1. Anlage zum Behandeln von dicht verschlossenen Lebensmittel-Behältnissen durch Berieselung mit einer zumindest ein Biozid enthaltenden Behandlungsflüssigkeit in zumindest einer mit einer Vielzahl von Berieselungsmitteln (B) zur Berieselung der Behältnisse ausgestatteten Behandlungszone (Z1-Z4), wobei die Anlage weiters Transportmittel zum Transportieren der Behältnisse durch die Behandlungszonen (Z1-Z4), einen Generator (G1) zur Herstellung einer Lösung des zumindest einen Biozids, zumindest eine Heizeinrichtung zum Erhitzen der Behandlungsflüssigkeit, Leitungen zum Zuführen der Biozid-Lösung vom Generator (G1) zu der zumindest einen Behandlungszone (Z1-Z4), und gegebenenfalls zu zumindest einer weiteren Biozid-Verbrauchereinrichtung (BV1, BV2), Leitungen zum Zuführen der Behandlungsflüssigkeit zu den Berieselungsmitteln (B), sowie die zur Steuerung der Flüssigkeitsströme erforderlichen Ventile (V1-V7), regelbaren Dosiereinrichtungen und Pumpen umfasst, **dadurch gekennzeichnet, dass**
a) die Anlage zumindest einen weiteren Generator (G2) zur Herstellung der Biozid-Lösung umfasst und
b) die Generatoren (G1, G2) weder direkt noch indirekt miteinander, aber über entsprechende Zuleitungen mit sämtlichen Behandlungszonen (Z1-Z4) und jeglichen weiteren Biozid-Verbrauchereinrichtungen (BV1, BV2) verbunden oder verbindbar sind.

2. Anlage nach Anspruch 1, **dadurch gekennzeichnet, dass** am Auslass jedes Generators (G1, G2) eine Rückschlagarmatur (R1, R2), vorzugsweise ein Rückschlagventil, vorgesehen ist.

3. Anlage nach Anspruch 1 oder 2, **dadurch gekennzeichnet, dass** die von den Generatoren (G1, G2) zu den Behandlungszonen (Z1-Z4) und jeglichen weiteren Biozid-Verbrauchereinrichtungen (BV1, BV2) führenden Zuleitungen über Mischventile (M3-M6) miteinander verbunden oder verbindbar sind.

4. Anlage nach einem der Ansprüche 1 bis 3, **dadurch gekennzeichnet, dass** die Behandlungsflüssigkeit eine wässrige Lösung des zumindest einen Biozids ist und die Generatoren (G1, G2) zur Herstellung einer wässrigen Lösung des zumindest einen Biozids mit variabler Konzentration dienen, die höher ist als die Biozid-Konzentration der Behandlungsflüssigkeit, und jeweils Zuleitungen für eine oder mehrere Biozid-Vorläuferlösungen aufweisen.

5. Anlage nach Anspruch 4, **dadurch gekennzeichnet, dass** die Generatoren (G1, G2) zur Herstellung einer wässrigen Chlordioxid-Lösung nach dem Peroxodisulfat-Chlorit-Verfahren dienen und jeweils Zuleitungen für wässrige Lösungen von Peroxodisulfat und von Chlorit aus entsprechenden Vorratsbehältern (P, C) aufweisen.

6. Anlage nach einem der Ansprüche 1 bis 5, **dadurch gekennzeichnet, dass** sie zur Pasteurisierung der in den Behältnissen eingeschlossenen Lebensmittel dient und zumindest eine Heizeinrichtung zum Erhitzen der Behandlungsflüssigkeit auf über 60 °C, vorzugsweise auf zumindest 80 °C, umfasst.

7. Anlage nach einem der Ansprüche 1 bis 6, **dadurch gekennzeichnet, dass** sie zumindest eine weitere Biozid-Verbrauchereinrichtung (BV1, BV2), ausgewählt aus Einrichtungen zum Spülen, Waschen, Befüllen und Verschließen der Behältnisse sowie zum Schmieren der Transportmittel, gegebenenfalls eines oder mehrerer Transportbänder, für die Behältnisse umfasst.

8. Anlage nach einem der Ansprüche 1 bis 7, **dadurch gekennzeichnet, dass** die Behandlungszonen (Z1-Z4) Teile eines Tunnelpasteurs, Plattenpasteurs oder Kammerpasteurs sind, der gegebenenfalls ein Tunnelpasteur ist.

9. Anlage nach einem der Ansprüche 1 bis 8, **dadurch gekennzeichnet, dass** sie weiters Messsensoren für die Temperaturen und/oder die Konzentrationen der Flüssigkeitsströme umfasst, die gegebenenfalls mit einer Steuereinheit zur Steuerung der Heizeinrichtungen, Dosiereinrichtungen, Pumpen und/oder Ventile verbunden sind.

10. Anlage nach einem der Ansprüche 1 bis 9, **dadurch gekennzeichnet, dass** sie weiters zumindest eine Mischkammer zum Vermischen der in zumindest einem Generator (G1, G2) hergestellten Biozid-Lösung mit Frischwasser und/oder zum Vermischen von zumindest zwei von unterschiedlichen Generatoren (G1, G2) hergestellten Biozid-Lösungen miteinander, und gegebenenfalls mit Frischwasser, vor deren Eintritt in die jeweilige Behandlungszone (Z1-Z4) oder in eine weitere Biozid-Verbrauchereinrichtung (BV1, BV2) umfasst.

11. Anlage nach einem der Ansprüche 1 bis 10, **dadurch gekennzeichnet, dass** sie weiters Folgendes umfasst:
eine oder mehrere Zuleitungen für Frischwasser, die gegebenenfalls mit einem Frischwasserreservoir (FR1, FR2) verbunden sind; und/oder
Mittel zum Sammeln und gegebenenfalls zum Rezyklieren von verbrauchter Behandlungsflüssigkeit; und/oder
weitere Heiz- oder Kühleinrichtungen; und/oder
Mittel zum Absaugen der die Behandlungszonen (Z1-Z4) umgebenden Atmosphäre.

12. Verfahren zum Behandeln von dicht verschlossenen Lebensmittel-Behältnissen durch Berieselung mit einer zumindest ein Biozid enthaltenden Behandlungsflüssigkeit unter Verwendung einer Anlage nach einem der Ansprüche 1 bis 11, wobei das Verfahren die folgenden Schritte umfasst:
das Herstellen einer Lösung des zumindest einen Biozids in zumindest zwei Generatoren (G1, G2), die weder direkt noch indirekt miteinander verbunden sind;
das Leiten der Biozid-Lösung zu zumindest einer mit einer Vielzahl von Berieselungsmitteln (B) ausgestatteten Behandlungszone (Z1-Z4), und gegebenenfalls zu zumindest einer weiteren Biozid-Verbrauchereinrichtung (BV1, BV2), wobei jeder Generator (G1, G2) mit sämtlichen Behandlungszonen (Z1-Z4) und jeglichen weiteren Biozid-Verbrauchereinrichtungen (BV1, BV2) verbunden oder verbindbar ist;
das Vermischen der Behandlungsflüssigkeit mit der Biozid-Lösung; und
das Berieseln der Behältnisse mit der Behandlungsflüssigkeit über die Berieselungsmittel (B), während sie durch die Behandlungszonen (Z1-Z4) transportiert werden.

13. Verfahren nach Anspruch 12, **dadurch gekennzeichnet, dass** die Behältnisse mit einer wässrigen Lösung des zumindest einen Biozids berieselt werden und in den Generatoren (G1, G2) eine wässrige Lösung des zumindest einen Biozids mit variabler Konzentration hergestellt wird, die höher ist als die Biozid-Konzentration der Behandlungsflüssigkeit,
wobei in den Generatoren (G1, G2) gegebenenfalls eine wässrige Chlordioxid-Lösung nach dem Peroxodisulfat-Chlorit-Verfahren aus Peroxodisulfat und Chlorit hergestellt wird.

14. Verfahren nach Anspruch 12 oder 13, **dadurch gekennzeichnet, dass** die in den Behältnissen eingeschlossenen Lebensmittel mit der Behandlungsflüssigkeit pasteurisiert werden, die vor dem Berieseln der Behältnisse auf über 60°C, gegebenenfalls auf zumindest 80 °C, erhitzt wird.

15. Verfahren nach einem der Ansprüche 12 bis 14, **dadurch gekennzeichnet, dass** die Biozid-Konzentration der in den Generatoren (G1, G2) hergestellten Biozid-Lösungen und der Behandlungsflüssigkeit gemessen werden und die der zumindest einen Behandlungszone (Z1-Z4) und gegebenenfalls weiteren Biozid-Verbrauchereinrichtungen (BV1, BV2) zugeführten Mengen der jeweiligen Flüssigkeitsströme anhand der gemessenen Werte gesteuert werden.

16. Verfahren nach einem der Ansprüche 12 bis 15, **dadurch gekennzeichnet, dass** in den Generatoren (G1, G2) zumindest zeitweise wässrige Biozid-Lösungen mit unterschiedlichen Konzentrationen hergestellt werden,
wobei gegebenenfalls Biozid-Lösungen aus verschiedenen Generatoren (G1, G2), die unterschiedliche Biozid-Konzentrationen aufweisen, vor ihrem Eintritt in eine Behandlungszone (Z1-Z4) und/oder in eine weitere Biozid-Verbrauchereinrichtung (BV1, BV2) miteinander vermischt werden.

17. Verfahren nach einem der Ansprüche 12 bis 17, **dadurch gekennzeichnet, dass** in die einzelnen Berieselungsmittel (B) einer Behandlungszone (Z1-Z4) Behandlungsflüssigkeit mit unterschiedlichen Temperaturen eingeleitet wird,
wobei gegebenenfalls in die in der Mitte einer Behandlungszone (Z1-Z4) gelegenen Berieselungsmittel (B) Behandlungsflüssigkeit mit der höchsten Temperatur eingeleitet wird, woraufhin sie sich während der Berieselung der Lebensmittel-Behältnisse abkühlt, unterhalb der Behältnisse gesammelt und rezykliert wird, indem sie in die am Anfang und am Ende der Zone gelegenen Berieselungsmittel (B) eingeleitet wird,
wobei die Behandlungsflüssigkeit mit der in den Generatoren (G1, G2) hergestellten Biozid-Lösung gegebenenfalls erst vermischt wird, sobald sie sich beim Berieseln der Behältnisse auf eine vorbestimmte Temperatur abgekühlt hat.

18. Verfahren nach einem der Ansprüche 12 bis 17, **dadurch gekennzeichnet, dass** kontinuierlich oder intermittierend Frischwasser zugeführt wird und/oder die die Behandlungszonen (Z1-Z4) umgebende Atmosphäre abgesaugt wird.
